# EUROPEAN PATENT APPLICATION

(11) **EP 2 732 821 A1**
(43) Date of publication of application: **21.05.2014**
(21) Application number: 12192633.1
(22) Date of filing: 14.11.2012
(51) Int. Cl.: A61K 31/785, A61K 31/787, A61P 33/06

(54) **Amphoteric polyamidoamines in the treatment of malaria**

(71) Applicant: Universita' Degli Studi Di Milano, 20122 Milano (IT); Fundació Privada Institut de Bioenginyeria de Catalunya, 08028 Barcelona (ES); Centre de Recerca en Salut Internacional de Barcelona, 08036 Barcelona (ES)
(72) Inventor: Ranucci, Elisabetta, 20122 MILANO (IT); Ferruti, Paolo, 20122 MILANO (IT); Fenili, Fabio, 20122 MILANO (IT); Manfredi, Amedea, 20122 MILANO (IT); Mauro, Nicolò, 20122 MILANO (IT); Fernández-Busquets, Xavier, 08036 BARCELONA (ES); Urban, Patricia, 08036 BARCELONA (ES)
(74) Representative: Barchielli, Giovanna

(57) **Abstract**

The present invention relates to the use of amphoteric polyamidoamines with MW of 10-100 kDa as antimalarial agents or carriers of antimalarial drugs and to formulations thereof.

## Description

The present invention relates to the use of polyamidoamines as antimalarial agents or carriers of antimalarial drugs and to formulations thereof.

Malaria is one of the most serious and complex health problems affecting humanity, despite the work of the research community towards its eradication. Malaria is an infectious disease caused by four species of the protozoan parasite *Plasmodium, P. falciparum* being the most severe of the four. All attempts to develop completely protective vaccines against *P. falciparum* have failed so far. Therefore, therapies and preventive measures against malaria are based on drugs. Various classes of antimalarial drugs exist such as chloroquine sulphadoxine/pyrimethamine combination. These drugs have side effects and inconvenient dosing schedules which limit the compliance of patients. Moreover resistance to many of the currently available antimalarial drugs is spreading rapidly, threatening people in areas where malaria is endemic.

Nanovectors, such as liposomes, solid-lipid nanoparticles, nano and microemulsions have been receiving attention to minimize the side effects of drug therapy. In vitro and in vivo tests on immunoliposomes generally indicate that they selectively direct chloroquine towards *Plasmodium*-infected red blood cells (pRBCs) (M. Owais et al., Antimicrob Agents Chemother., 39, 180-184, 1995). Liposomes can also prolong the duration of chloroquine exposure, acting as a slow-release reservoir. However, little data are available on liposome-driven antimalarial drug targeting to human-infecting parasites. Limited control over drug release, low encapsulation capacity, poor shelf stability, short in vivo circulation times and difficult formulation for oral administration are some of the drawbacks of liposomes.

Biodegradable polymeric nanoparticles have shown a potential to increase the bioavailability of insoluble antimalarial drugs. Several polymeric nanocarriers have been proposed to deliver antimalarial drugs *in vivo,* including quinine, halofantrine, curcumin, artemisinin derivatives, chloroquine, and primaquine. However, only few cases in the literature describe in malaria-infected mice the efficacy of polymer-encapsulated drugs compared with the free compounds. Orally administered lipid nanoemulsions of primaquine at a dose of 1.5 mg kg⁻¹ day⁻¹ improved the mean survival time of *P*. *berghei-*infected mice from 13 to 39 days, and reduced parasitemia 4-fold relative to free drug (Singh, K.K. et al. International Journal of Pharmaceutics 347, 136-143, 2008); oral delivery of curcumin bound to chitosan nanoparticles cured all *P*. *yoelii-*infected mice when administered at 100 mg kg-¹, whereas the same amount of the free drug cured only one third of the animals (Akhtar, F. et al. Biotechnology Advances 30, 310-320, 2012); intravenous administration to *P*. *berghei-*infected rats of several doses of 75 mg kg⁻¹ day⁻¹ quinine enclosed in nanocapsules prepared with poly(*ε*-caprolactone) and Polysorbate 80 improved by 30% the efficacy of free quinine (Haas, S.E. et al. International Journal of Antimicrobial Agents 34[2], 156-161, 2009); finally, a single intravenous dose of 1 mg kg⁻¹ halofantrine encapsulated in poly(D,L-lactide)-based polymer cured 80% of *P*. *berghei-*infected mice, when the same dose of the free drug was unable to arrest the infection or to prolong the survival of the animals (Mosqueira, V.C.F. et al. Antimicrobial Agents and Chemotherapy 48, 1222-1228, 2004).

Therefore the need remains of having anti-malarial drugs with increased activity, lower side effects and less prone to induce *Plasmodium* resistance.

It has now unexpectedly been found that particular polyamidoamines (PAA) with amphoteric properties have antimalarial activity per se. Furthermore it has been found that said PAA and their conjugates with proteins are able to carry anti-malarial drugs into infected red blood cells. PAAs have been observed to increase the activity of the drugs.

Object of the present invention are amphoteric PAA having a MW of 10-100 kDa, for use in the treatment and/or prevention of malaria.

Malaria may be caused by different species of *Plasmodium,* in particular *Plasmodium falciparum, Plasmodium vivax, Plasmodium ovale, Plasmodium malariae,* and *Plasmodium knowlesi.*

Preferred fractions are those having a MW of 10-30 kDa, 30-50 kDa, 50-100 kDa. Most preferred is the fraction having a MW of 50-100 kDa.

The amphoteric PAA for the use of the present invention have the following formulae (I), (II) or (III): 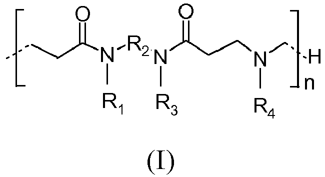 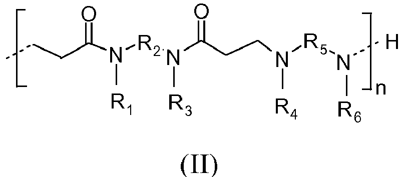 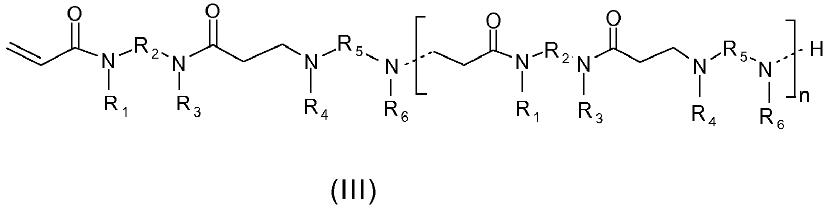 wherein R₁ and R₃, which may be the same or different, are H or a group C₁₋₆ alkyl, optionally substituted with a group -COOH, -SO₃H, -OPO₃H, -OH, or -N(R₇,R₈) or -N(R₇,R₈, R₉), in which anyone of R₇, R₈ and R₉ independently is a group C₁₋₄ alkyl;
R₄ and R₆, which may be the same or different, are a group C₁₋₆ alkyl, optionally substituted with a group -COOH, -SO₃H,-OPO₃H, -OH, -N(R₇,R₈) or -N(R₇,R₈, R₉)⁺, or -HN-C(NH₂)=NH, in which R₇, R₈ and R₉ are as above defined;
R₂ and R₅, which may be the same or different, are C₁-C₄ alkylene, optionally substituted with a group -(CH₂)ₘ-X, wherein m is an integer between 0 and 6 and X is a group chosen among -COOH, -SO₃H, -OPO₃H, -OH, or -N(R₇,R₈) or -N(R₇,R₈, R₉)⁺ wherein R₇,R₈ and R₉ are as above defined;
or the groups R₁, R₂ and R₃, or the group R₄, R₅ and R₆ together with the two adjacent nitrogen atoms form a saturated heterocyclic group, optionally substituted with a C₁₋₄ alkyl group.
n is an integer expressing the number of repeating units. The value of n is such as to give the reported molecular weight of the polymer.

Preferably R₁, R₃, are H.

Preferably R₂ is -CH₂-, -CH(COOH)- or R₁, R₂ e R₃, together with the two adjacent nitrogen atoms form a saturated heterocyclic group:

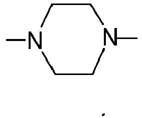

The term amphoteric PAA means PAA containing both basic and acidic groups in their polymer backbone.

The amphoteric PAA of formula (I) or (II) and the methods for their preparation are disclosed in WO 2010/099962, WO2011/145056 and in Ferruti P. et al., Biomacromolecules: 2007,8,1498-1504.

Amphoteric PAA of Formula (III) and methods for their preparation are disclosed in the Italian co-pending application IT MI2012A000953 and in Ferruti et al. Journal of Polymer Chemistry: 2012, DOI: 10.1002/pola.26325.

The most preferred amphoteric PAA for the use of the present invention are:

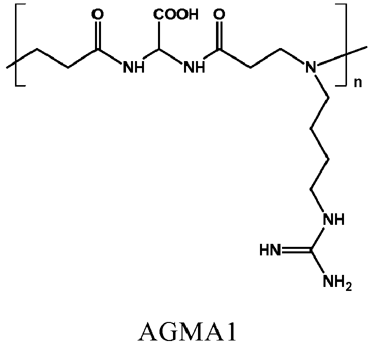

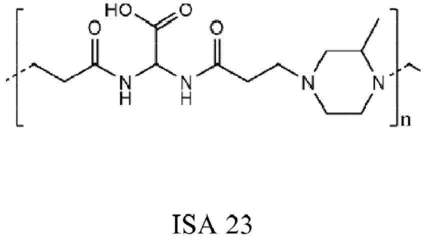

The amphoteric PAA of the invention are able to target red blood cells infected by *Plasmodium falciparum*; AGMA1, in addition, shows a significant antimalarial activity. Therefore the amphoteric PAA being biocompatible and biodegradable polymers, are a new class of antimalarial agents with reduced side effects and might be less prone to induce *Plasmodium* resistance than other targeting agents whose ligands are small antigenic regions.

The amphoteric polyamidoamine bearing guanidine residues in its main chain named AGMA1, reported above, obtained by polyaddition of 2,2-bisacrylamiodacetic acid and 4-aminobutylguanidine, is a well-known bioactive and biodegradable polymer, with an IC₅₀>5 mg/ml and MTD>0.5 g/Kg in mice.

AGMA1 is known to play a role in the integrin-mediated adhesion to the extracellular matrix. In fact, it acts as adhesion promoter of several primary cells, including neuronal cells (WO2010/099962). AGMA1 inhibits both HSV-1 and HSV-2 infection within the nanomolar range in vitro. It exerts its antiviral activity in vitro even when administered before virus infection. It is resistant to acidic pH like that found in vagina and does not induce inflammatory response and proves its antiviral activity in vivo.

It has now been found that AGMA1 has a significant antimalarial activity.

Another object of the present invention are the amphoteric PAA having a MW of 10-100 kDa, as defined above, as carrier of antimalarial drugs and the compositions comprising the amphoteric PAA as defined above and antimalarial drugs and their use in malaria treatment and/or prevention.

The anti malarial drugs can be selected from the group consisting of quinine and quinidine, atovaquone, chloroquine and hydroxychloroquine, mefloquine, primaquine, artemisinin and their derivatives.

The compositions containing an amphoteric PAA and an antimalarial drug show a synergistic effect in comparison to the sum of the effects of PAA and antimalarial drug alone.

A further object of the present invention are the conjugates of the amphoteric PAA of Formula (III) having a MW of 10-100 kDa, preferably 50-100 kDa as defined above, with proteins for use as carrier of antimalarial drugs and the compositions comprising conjugates of the amphoteric PAA of Formula (III) with protein as reported above and antimalarial drugs and their use in the treatment and/or prevention of malaria. The protein is preferably an albumin or acid alpha-1-glycoprotein.

The conjugates are obtained by reaction of the terminal acrylamide group of the PAA with SH or amine group present in the protein. Preferred proteins are albumins and acid alphal-glycoprotein. The conjugates and their preparation are disclosed in Ferruti et al. Journal of Polymer Science DOI: 10.1002/pola.26325 and in the co-pending application IT MI2012A000953. Said conjugates have a high binding ability towards antimalarial drugs coupled with localization inside red blood cells infected with *Plasmodium.* Particularly preferred are the conjugates of ISA23 with albumin obtained as reported in the below Scheme 1.

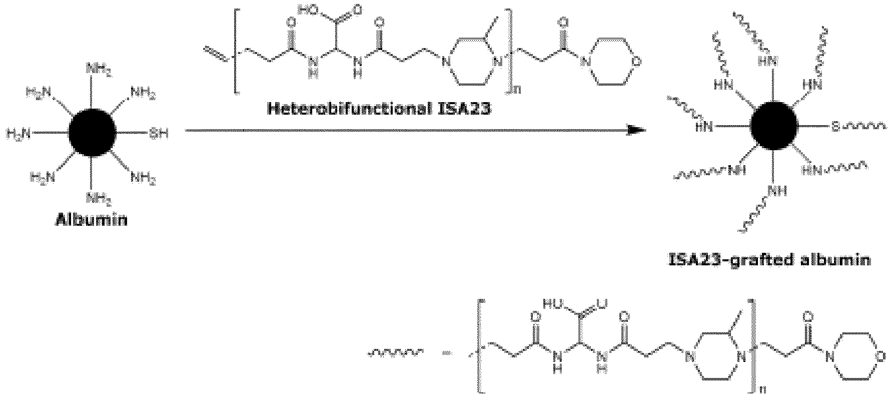

The amphoteric PAA as described above, the compositions comprising the amphoteric PAA and an antimalarial drug as described above and the composition comprising the conjugates of an amphoteric PAA with a protein and an antimalarial drug as described above may be formulated with conventional carriers and excipients, which will be selected in accord with ordinary practice.

Another object of the invention is a method for blocking transmission of malaria parasites comprising applying an amphoteric polyamidoamine as defined above or a composition comprising an amphoteric PAA and an antimalarial drug as defined above or a composition comprising a conjugates of an amphoteric PAA with a protein and an antimalarial drug as described above to mosquitoes, in particular by feeding or spraying.

Excipients may include inter alia surfactants, preservatives, antioxidants, chelating agents, carbohydrates, hydroxyalkylcellulose, hydroxyalkylmethyl-cellulose, poloxamer, polymeric stabilizing agents and the like. The pharmaceutical compositions include those suitable for different administration routes, such as for example oral, intravenous, and parenteral. The pharmaceutical compositions may conveniently be presented in unit dosage form and may be prepared by any of the methods well known in the art of pharmacy. When used for oral use for example, tablets, troches, lozenges, aqueous or oil suspensions, dispersible powders or granules, emulsions, hard or soft capsules, syrups or elixirs may be prepared. Compositions intended for oral use may be prepared according to any method known to the art for the manufacture of pharmaceutical compositions and such compositions may contain one or more agents including sweetening agents, flavoring agents, coloring agents and preserving agents, in order to provide a palatable preparation. Compositions suitable for parenteral administration include aqueous and non-aqueous sterile injection solutions or suspensions containing the active ingredient(s).

The daily dosage of the polymer may vary from 100 to 1000 mg, preferably from 200 to 600 mg. The dosage may be adjusted on the basis of efficacy, pharmaco-kinetic and toxicological studies. The dosage of anti-malarial drug is that used for the drug alone or a lower dose.

### Examples

### Example 1: Synthesis of AGMA1

Linear AGMA1 was prepared by a procedure similar to that previously reported (Franchini et al., Biomacromolecules, 2006, 7, 1215-1222). After that AGMA1 was ultrafiltered through membranes with nominal cut-off 100000, 50000, 30000, 10000, 5000, 1000, in order to obtain four AGMA1 with the following molecular weight range: 100000-50000, 30000-10000, 10000-5000, and 5000-1000.

### Example 2: Synthesis of ISA23

Four linear ISA23 were synthesized as previously described (Richardson et al., J. Drug Targeting, 1999, 6 (6), 391-404). The crude reaction was ultrafiltered through membranes with nominal cut-off 100000, 30000, 10000, 3000, 1000, in order to obtain AGMA1 with the following molecular weight range: 100000-30000, 30000-10000, 10000-3000, and 3000-1000.

### Example 3: Preparation of the formulations ISA 23 - CHLOROQUINE/PRIMAQUINE SALTS

ISA23 was prepared as above reported (Mn=29800; PD=1.28). ISA23 (48% w/w aqueous solution, pH 10-11) was brought to its isoelectric point (I.P.) (pH 5.2) by adding the exact amount of HCl(0.27 mL HCl_{conc} /g ISA23) necessary to acidify the COO-Li⁺ equivalents borne in the repeating units of ISA23 as present in the polymerization mixture. The crude polymerization mixture was then ultrafiltered through a membrane with molecular weight cut-off 5000. The final product was retrieved by freeze-drying. It should be observed that ultrafiltration allows removing practically all LiCl produced by acidification of ISA23, as ascertained by AgCl precipitation assay, which revealed the presence of only traces of Cl⁻.

A 0.1 mg/mL methanol solution of PQ (or CQ) free base was added drop-wise to a 10 mg/mL ISA23 solution at I.P. (pH 5.2) until pH 7-7.4 was obtained. The resultant clear solution was freeze-dried and the polymer salt retrieved as a white powder.

### Example 4: Preparation of the formulations AGMA 1- CHLOROQUINE/PRIMAQUINE SALTS

AGMA1 was prepared as above reported (Mn=20800; PD=1.38). A 10 mg/mL aqueous solution of AGMA1 pH 5.5 was first prepared. A 0.1 mg/mL methanol solution of PQ (or CQ) free base was added drop-wise to this solution until pH 7-7.4 was reached. The resultant clear solution was freeze-dried and the product recovered as a white solid.

### Example 5. Antimalarial activity of AGMA1 and ISA23

AGMA1 and ISA 23 samples (obtained as reported in examples 1 and 2) with different molecular weights were tested as antimalarial agent in *in vitro* growth inhibition assays (GIAs). Parasitemia of *P. falciparum* 3D7 cultures was adjusted to 1.5% with more than 90% of parasites at ring stage after sorbitol synchronization. 98 µl of this *Plasmodium* culture was plated in 96-well plates and incubated in the presence of each polymer solution for 48 h at 37°C under a gas mixture of 92% N₂, 5% CO₂, and 3% O₂. Parasitemia was determined by microscopic counting of blood smears, or by fluorescence-assisted cell sorting (FACS).

Results are reported in Table 1.

Inhibition of *P. falciparum* growth increased with polymer size, in good correlation with the increased binding of higher molecular mass AGMA1 to pRBCs observed in FACS analysis. The highest antimalarial AGMA1 activity corresponded to the 50-100-kDa fraction, which at a concentration of 1 mg/mL (10 µM for a 100-kDa polymer) reduced parasitemia by 81.5%. AGMA1 IC50 in *P. falciparum* growth inhibition assays is around 0.25 mg/mL for the 50-100 kDa fraction. The 30-100 kDa ISA23 fraction was observed to reduce parasitemia by 34.8% at 2 mg/mL.

**Table 1. Average and standard deviation of Plasmodium growth inhibition using different molecular weight fractions of AGMA1 and ISA 23 polymers. The values express percentage of reduction respective to the parasitemia of controls.**

| AGMA 1 (10-30K) | % inhibition | SD | | AGMA 1 (50-100K) | % inhibition | SD |
|---|---|---|---|---|---|---|
| 1 mg/mL | 65.4 | 8.74 | | 1 mg/mL | 81.5 | 11.96 |
| 0.5 mg/mL | 54.8 | 9.87 | | 0.5 mg/mL | 73.2 | 9.90 |
| 0.25 mg/mL | 25.2 | 9.99 | | 0.25 mg/mL | 50.3 | 9.61 |
| 0.125 mg/mL | 2.2 | 3.91 | | 0.125 mg/mL | 9.3 | 5.47 |

| ISA 23 (10-30K) | % inhibition | SD | | ISA 23 (50-100K) | % inhibition | SD |
|---|---|---|---|---|---|---|
| 2 mg/mL | 17.5 | 3.4 | | 2 mg/mL | 34.8 | 5.9 |
| 1 mg/mL | 12.5 | 1.8 | | 1 mg/mL | 16.3 | 2.3 |
| 0.5 mg/mL | 5.4 | 1.1 | | 0.5 mg/mL | 8.5 | 1.9 |
| 0.25 mg/mL | 1.8 | 0.6 | | 0.25 mg/mL | 2.5 | 2.1 |

### Example 6. Specific targeting of ISA23 and AGMA 1 to infected RBCs

FITC-labelled AGMA1 (Mn=15500, PD=1.24) was prepared following the procedure previously described (Ferruti, P. et al., Biomacromolecules 2007, 8 (5), 1498-1504). FITC-labelled ISA23 (Mn=12100, PD=1.56) was prepared by treating with a FITC solution in methanol (0.2 mg/mL) a 10 mg/mL solution in buffer pH 7.4 of an ISA23 sample (ISA23-NH₂) carrying amine groups as side substituents. In turn, ISA23-NH₂ was obtained by substituting 7% on a molar basis of mono(*tert-*butoxycarbonyl)ethylenediamine for 2-methyl-piperazine. The mixture was stirred overnight at room temperature and then centrifuged to eliminate insoluble impurities. The resultant clear solution was then dialyzed and the fluorescein-labeled polymer isolated by freeze-drying the retained portion. The recovery was quantitative. The conjugation of ISA23-NH₂ with FITC was confirmed by NMR and fluorescence microscopy and the efficiency of the labelling procedure was determined by measuring the fluorescence intensity at λₑₓ=480 nm and λₑₓ=520 nm of a solution of

ISA23-FITC versus a standard FITC solution of known concentration.

Confocal fluorescence microscopy and flow cytometry were used to study the targeting of ISA23 or AGMA1 to infected RBCs.

Fluorescein-labeled polymer at a concentration of 0.3 mg/mL was added to *P.falciparum* cultures of the 3D7 strain or to *P.yoelii*-infected mouse blood, and after 90 min of incubation polymer binding was detected by confocal fluorescence and FACS.

Targeting analysis done by FACS indicated that FITC-labeled AGMA1 (Mn=15500) and ISA23 (Mn=12100) polymers specifically target pRBCs. The specificity of targeting observed by FACS was also examined by confocal fluorescence microscopy analysis of living pRBC cultures treated with FITC-labeled polymers. Both AGMA1 and ISA23 specifically target pRBCs infected by the *P. falciparum* late forms trophozoites and schizonts, but not ring-infected pRBCs. After 90 min both polymers are detected inside target cells, in the close vicinity of DAPI-stained *Plasmodium* DNA (this result has been confirmed by transmission electron microscopy). This targeting to pRBCs and to the parasite itself can also be observed for the mouse malaria parasite *P. yoelii,* where the binding of ISA23 to merozoites (free *Plasmodium* parasites) is particularly striking. This result offers interesting perspectives for PAAs as targeting elements not only towards pRBCs but also towards free merozoites.

### Example 7. In vitro antimalarial activity of compositions of PAA and chloroquine or primaquine.

Parasitemia of *P. falciparum* 3D7 cultures was adjusted to 1.5% with more than 90% of parasites at ring stage after sorbitol synchronization. 98 µL of this *Plasmodium* culture was plated in 96-well plates and incubated in the presence of phosphate-buffered saline (PBS) solutions of the free drugs or of polymer/drug conjugates (ISA 23-PQ obtained in Example3; AGMA1-PQ obtained in Example 4,; ISA 23-CQ obtained in Example 3; AGMA1-CQ obtained in Example 4) for 48 h at 37°C under a gas mixture of 92% N₂, 5% CO₂, and 3% O₂. Parasitemia was determined by microscopic counting of blood smears, or by FACS.

**Table 2. Effect of free primaquine, ISA23-PQ and AGMA1-PQ on the growth of P. falciparum. The polymers were added to P. falciparum cultures at the ring stage, and incubated for 48 h before determining parasitemia.**

| PQ concentration | % inhibition PQ soluble | SD inhibition PQ soluble | % inhibition ISA23-PQ | SD inhibition ISA23-PQ | % inhibition AGMA1-PQ | SD inhibition AGMA1-PQ |
|---|---|---|---|---|---|---|
| 14.1 µg/mL | 88.2 | 0.6 | 91.5 | 0.2 | 93.5 | 0.4 |
| 7.04 µg/mL | 86.6 | 0.5 | 88.3 | 2.4 | 89.7 | 1.7 |
| 3.52 µg/mL | 80.3 | 1.1 | 77.6 | 5.1 | 86.0 | 3.3 |
| 1.76 µg/mL | 53.9 | 1.4 | 48.2 | 4.3 | 68.9 | 7.7 |
| 0.88 µg/mL | 40.9 | 2.4 | 23.3 | 5.6 | 37.5 | 11.5 |
| 0.44 µg/mL | 6.9 | 1.2 | 12.1 | 2.9 | 8.2 | 1.8 |

**Table 3. Effect of free chloroquine, ISA23-CQ and AGMA1-CQ on the growth of P. falciparum. The polymers were added to P. falciparum cultures at the ring stage, and incubated for 48 h before determining parasitemia.**

| CQ concentration | % inhibition CQ soluble | SD inhibition CQ soluble | % inhibition ISA23-CQ | SD inhibition ISA23-CQ | % inhibition AGMA1-CQ | SD inhibition AGMA1-CQ |
|---|---|---|---|---|---|---|
| 82.4 ng/mL | 86.4 | 1.3 | 91.7 | 1.1 | 84.3 | 1.0 |
| 41.4 ng/mL | 85.9 | 1.5 | 90.4 | 0.4 | 88.5 | 1.7 |
| 20.6 ng/mL | 82.1 | 0.9 | 89.5 | 1.7 | 88.1 | 1.4 |
| 10.3 ng/mL | 72.9 | 1.7 | 49.3 | 2.9 | 74.6 | 6.2 |
| 5.15 ng/mL | 22.6 | 0.7 | 14.5 | 1.5 | 23.4 | 2.5 |
| 2.6 ng/mL | 8.9 | 1.0 | 8.0 | 0.7 | 6.9 | 2.1 |

In standard GIAs, the non-covalent conjugation of AGMA1 and ISA23 with the antimalarial drugs primaquine (AGMA1-PQ, ISA23-PQ) and chloroquine (AGMA1-CQ, ISA23-CQ) increased slightly *the in vitro* antimalarial activity of the drugs. Covalent conjugation, on the other hand, significantly decreased drug efficacy (data not shown). Addition of drugs and PAA-drug conjugates at trophozoite stage in a modified GIA or addition to either ring or trophozoite stage for 1 h before removing the drugs and incubating for a further 48 h before parasitemia determination improved the efficacy of the encapsulated drugs relative to their free forms. These results suggest a potentiation of PAA-drug intake by pRBCs, a result particularly remarkable for CQ, which has an endogenous carrier across human erythrocyte membranes that accumulates the drug selectively in these cells.

### Example 8. In vivo experiments with PAAs-chloroquine

The *in vivo* antimalarial activity of free chloroquine, ISA23-CQ and AGMA1-CQ (obtained as reported above) was analyzed by using a 4-day-blood suppressive test as previously described (Fidock, D.A. et al. Nat. Rev. Drug Discov. 3, 509-520, 2004). Briefly, mice were inoculated 2 × 10⁶ red blood cells from *P. yoelii* XL-infected mice by intraperitoneal (ip) injection. The chemotherapy treatment started 2 hours later (day 0) with a single dose of 1 mg kg⁻¹ day⁻¹ chloroquine (n=3) administered as free chloroquine, ISA23-CQ and AGMA1-CQ by an ip injection followed by identical dose administration for the following 3 days. Tested compounds were prepared at appropriate doses in PBS. The control groups received PBS (n=3). The parasitemia was monitored daily by microscopic examination of Wright's-stained thin-blood smears. Activity was calculated by microscopic counting of blood smears from day 4.

**Table 4. Results of activity on day 4 in a 4-days suppressive test with malaria-infected mice of free chloroquine, and PAA-CQ formulations.**

| Sample | % parasitemia | Activity (% parasitemia reduction) |
|---|---|---|
| Control (PBS) | 44.8 | |
| CQ 1 mg/kg | 37.7 | 15.8 ± 3.8 |
| CQ 2.5 mg/kg | 20.1 | 55.1 ± 6.6 |
| AGMA1-CQ 1 mg/kg | 1.6 | 96.5 ± 2.4 |
| ISA23-CQ 1 mg/kg | 7.4 | 83.5 ± 7.8 |

In a 4-day suppressive test (Table 4), *P*. *yoelii*-infected mice were freed of parasites and cured after intraperitoneal administration of 4 × 1 mg kg⁻¹ doses of CQ encapsulated in AGMA1 or ISA23, whereas the same or even a larger amount of free CQ was unable to eliminate parasitemia or cure the animals.

The *in vitro* activity of encapsulated CQ was modest in comparison with its *in vivo* performance. This result could be explained because the random encounters between pRBCs and polymers required for targeting specificity to be manifested are favored by the turbulent mixing in the blood circulation. Alternatively, drug release could be facilitated by some blood factor triggering polymer breakdown. *In vitro,* good PAA-mediated targeting might cancel out with slow drug release from the polymers, resulting in no clear increase in encapsulated drug efficacy *νs.* its free form.

AGMA1 and ISA23 intrinsic pRBC targeting without the need for including bulky and often expensive ligands will contribute to the design of smaller nanovectors, apt for oral formulation, and of lower cost, a desirable characteristic when designing medicines to be used in developing areas with reduced per capita incomes.

## Claims

1. An amphoteric polyamidoamine having a MW of 10-100 kDa for use in the treatment and/or prevention of malaria.

2. An amphoteric polyamidoamine having a MW of 30-50 kDa for use according to claim 1.

3. An amphoteric polyamidoamine having a MW of 50-100 kDa for the use according to claim 1.

4. The amphoteric polyamidoamine for the use according to each one of claims 1-3 which have the following formulae (I), (II) or (III): 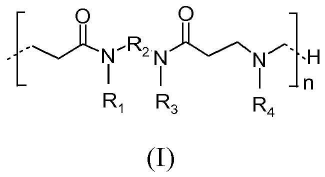 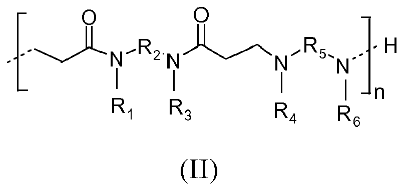 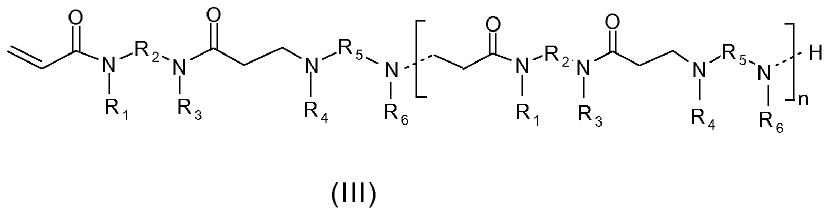 wherein R₁ and R₃, which may be the same or different, are H or a group C₁₋₆ alkyl, optionally substituted with a group -COOH, -SO₃H, -OPO₃H, -OH, or -N(R₇,R₈) or -N(R₇,R₈, R₉), in which anyone of R₇, R₈ and R₉ independently is a group C₁₋₄ alkyl;
R₄ and R₆, which may be the same or different, are a group C₁₋₆ alkyl, optionally substituted with a group -COOH, -SO₃H,-OPO₃H, -OH, -N(R₇,R₈) or -N(R₇,R₈, R₉)⁺, or -HN-C(NH₂)=NH, in which R₇, R₈ and R₉ are as above defined; R₂ and R₅, which may be the same or different, are C₁-C₄ alkylene, optionally substituted with a group -(CH₂)ₘ-X, wherein m is an integer between 0 and 6 and X is a group chosen among -COOH, -SO₃H, -OPO₃H, -OH, or -N(R₇,R₈) or -N(R₇,R₈,R₉)⁺ wherein R₇,R₈ and R₉ are as above defined;
or the groups R₁, R₂ e R₃, or the group R₄, R₅ and R₆ together with the two adjacent nitrogen atoms form a saturated heterocyclic group, optionally substituted with a C₁₋₄ alkyl group.

5. The amphoteric polyamidoamine for the use according to claim 4 wherein R₁ and R₃, are H, R₂ is -CH₂-, -CH(COOH)- or R₁, R₂ and R₃, together with the two adjacent nitrogen atoms form a saturated heterocyclic group of formula:

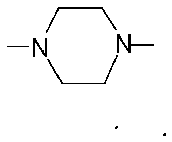

6. The amphoteric polyamidoamine for the use according to claim 1 having the formula:

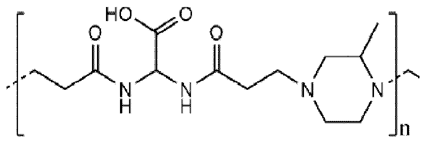

7. The amphoteric polyamidoamine for the use according to claim 1 having the formula:

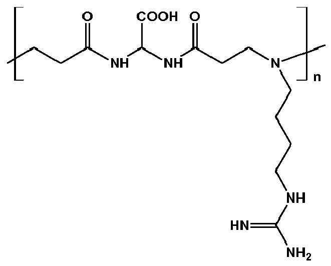

8. The amphoteric polyamidoamine for the use according to any one of claims 1-7 wherein malaria is caused by *Plasmodium falciparum, Plasmodium vivax, Plasmodium ovale, Plasmodium malariae,* and *Plasmodiumknowlesi.*

9. A pharmaceutical composition comprising an amphoteric polyamidoamine as defined in claims 1-7 and a pharmaceutically acceptable excipient and/or carrier.

10. An amphoteric polyamidoamine as defined in claims 1-7 for use as carrier of an antimalarial drug.

11. A composition comprising an amphoteric polyamidoamine as defined in claims 1-7 and an antimalarial drug.

12. The composition according to claim 11 wherein the antimalarial drug is selected from the group consisting of quinine and quinidine, atovaquone, chloroquine and hydroxychloroquine, mefloquine, primaquine, artemisinin and their derivatives.

13. The composition according to claim 11 or 12 for use in the treatment and/or prevention of malaria.

14. A conjugate of an amphoteric PAA of Formula (III) having a MW of 10-100 kDa as defined in claim 1 with a protein for use as carrier of antimalarial drugs.

15. A composition comprising a conjugate of the amphoteric PAA of Formula (III) having a MW of 10-100 kDa as defined in claim 1 with a protein and an antimalarial drug.

16. The composition according to claim 15 wherein the protein is an albumin or acid alpha-1-glycoprotein.

17. The composition of claim 15 or 16 for use in the treatment and/or prevention of malaria.

18. A method for blocking transmission of malaria parasites comprising applying a polyamidoamine as defined in claims 1-7 or a composition as defined in claim 11 or 12 or a composition as defined in claim 15 or 16 to mosquitoes.

19. The method according to claim 18 wherein a polyamidoamine as defined in claims 1-7 or a composition as defined in claim 11 or 12 or a composition as defined in claim 15 or 16 is applied to mosquitoes by feeding or spraying.
